(19) Europäisches Patentamt
European Patent Office
Office européen des brevets

(11) EP 2 043 991 B1

(12) **FASCICULE DE BREVET EUROPEEN**

(45) Date de publication et mention de la délivrance du brevet:
**31.08.2011 Bulletin 2011/35**

(21) Numéro de dépôt: **07788928.5**

(22) Date de dépôt: **22.06.2007**

(51) Int Cl.:
***C07C 45/50*** (2006.01) ***C07C 47/02*** (2006.01)

(86) Numéro de dépôt international:
**PCT/FR2007/001052**

(87) Numéro de publication internationale:
**WO 2008/006951 (17.01.2008 Gazette 2008/03)**

(54) **PROCEDE D'HYDROFORMYLATION METTANT EN OEUVRE UN CATALYSEUR A BASE DE COBALT DANS UN LIQUIDE IONIQUE NON-AQUEUX**

HYDROFORMYLIERUNGSVERFAHREN MIT EINEM KATALYSATOR AUF KOBALTBASIS IN EINER NICHTWÄSSRIGEN IONENFLÜSSIGKEIT

HYDROFORMYLATION PROCESS USING A COBALT-BASED CATALYST IN A NONAQUEOUS IONIC LIQUID

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **13.07.2006 FR 0606497**

(43) Date de publication de la demande:
**08.04.2009 Bulletin 2009/15**

(73) Titulaire: **IFP Energies nouvelles**
**92852 Rueil-Malmaison Cedex (FR)**

(72) Inventeurs:
- **MAGNA, Lionel**
**F-69007 Lyon (FR)**
- **SAUSSINE, Lucien**
**F-78290 Croissy sur Seine (FR)**
- **PRORIOL, David**
**F-69530 Brignais (FR)**
- **OLIVIER-BOURBIGOU, Hélène**
**F-69230 St Genis Laval (FR)**

(56) Documents cités:
**EP-A1- 1 106 595 FR-A1- 2 838 431**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 043 991 B1

## Description

La présente invention concerne un procédé d'hydroformylation de composés oléfiniquement insaturés au moyen d'un catalyseur à base de cobalt mis en oeuvre dans un liquide ionique non-aqueux, comprenant au moins un cation $Q^+$ et au moins un anion $A^-$ avec un recyclage du catalyseur amélioré.

L'hydroformylation des composés oléfiniques est une réaction de grande importance industrielle et la plupart des procédés ont recours à des catalyseurs homogènes dissous dans une phase organique constituée des réactifs, des produits et éventuellement d'un excès de ligand, si bien que l'on rencontre des difficultés pour séparer et récupérer le catalyseur, en particulier quand celui-ci est employé en relativement grande quantité, comme c'est le cas pour les procédés utilisant un catalyseur à base de cobalt.

Une solution en vue de résoudre ce problème a été évoquée par Bartik et al. : Organometallics (1993) 12 164-170, J. Organomet. Chem. (1994) 480 15-21, ainsi que par Beller et al. : J. Molecular Catal. A: Chemical (1999) 143 31-39. Elle consiste à effectuer l'hydroformylation en présence d'une solution aqueuse contenant un complexe du cobalt rendu hydrosoluble grâce à la présence d'un ligand phosphine-sulfonate, tel que le sel de sodium de la triphénylphosphine trisulfonée ou d'une tris-(alkylphényl)-phosphine trisulfonée. De cette manière, la phase organique contenant les aldéhydes est aisément séparée de la phase aqueuse contenant le catalyseur.

Malgré l'intérêt de ces différents systèmes, la faible capacité de l'eau à dissoudre certains substrats organiques comme des oléfines à longue chaîne, constitue une limitation majeure de ces procédés. L'hydroformylation de ce type de charge conduit très souvent à de faibles vitesses de réaction, rendant toute application industrielle inenvisageable. De plus, l'eau est un solvant protique très coordinant, qui peut être réactif vis-à-vis des catalyseurs. Bien qu'elle soit d'un très grand intérêt, l'utilisation de l'eau comme solvant de réaction n'est pas généralisable à tous les types de catalyseurs et de substrats.

Il a été décrit dans le brevet US-A-5 874 638 de la demanderesse que certaines limitations liées à l'utilisation de l'eau comme solvant de réaction (en particulier la solubilité des oléfines longues) peuvent être levées en dissolvant certains composés catalytiques des métaux de transition des groupes 8, 9 et 10, connus pour catalyser l'hydroformylation, dans des liquides ioniques non-aqueux qui sont constitués par des sels organiques-inorganiques liquides à température ambiante.

Cependant, lorsque le catalyseur comprend un sel ou un complexe du cobalt, il est très difficile d'empêcher la formation, au moins en partie, de dicobalt octacarbonyle et/ou d'hydrure de cobalt tétracarbonyle dans les conditions de la réaction d'hydroformylation. Ces deux composés étant bien solubles dans la phase organique de réaction constituée au moins par le réactif oléfinique et les aldéhydes produits, le recyclage du cobalt au moyen de la phase liquide ionique non-aqueux n'est que partiel, ce qui entraîne des pertes de catalyseur.

Par ailleurs, il a été montré dans le brevet US-A-6 617 474 de la demanderesse qu'il était possible d'augmenter les vitesses de réaction en réalisant la réaction dans un liquide ionique partiellement ou complètement miscible avec les produits de la réaction, tout en conservant le bénéfice de la séparation et de la réutilisation du liquide ionique contenant le catalyseur et en améliorant la récupération des produits de la réaction, en injectant après la section réactionnelle un solvant organique, peu ou pas miscible avec le liquide ionique, qui peut avantageusement être le composé oléfiniquement insaturé à hydroformyler et qui améliore la démixtion des produits de l'effluent réactionnel.

Dans ce contexte, il avait été trouvé et décrit dans la demande de brevet US-A-2003/0225303 de la demanderesse que, dans la réaction d'hydroformylation catalysée par des complexes du cobalt mis en oeuvre dans un liquide ionique non-aqueux, le recyclage du métal dans le liquide ionique était grandement amélioré par l'utilisation d'un ligand choisi parmi les bases de Lewis et simultanément par la mise en oeuvre d'une étape de dépressurisation intermédiaire entre l'étape de réaction sous pression et l'étape de séparation des phases par décantation. À la fin de cette étape de dépressurisation, la phase organique est séparée dans l'étape de décantation et la phase liquide ionique non-aqueux contenant le catalyseur peut être réutilisée.

Il a maintenant été trouvé qu'il était possible d'améliorer considérablement la vitesse de réaction, d'une part, et la rétention et le recyclage du catalyseur au cobalt dans la phase liquide ionique, d'autre part, par l'addition du ligand dans une étape post-réactionnelle. La présente invention propose donc une nouvelle mise en oeuvre du système visant notamment à allier une vitesse de réaction élevée avec une rétention et un recyclage du catalyseur améliorés.

Ainsi, le procédé d'hydroformylation en phase liquide de composés oléfiniquement insaturés de l'invention peut être défini en ce qu'il comprend :

- une étape réactionnelle, effectuée en présence d'au moins un liquide ionique non-aqueux comprenant au moins un sel de formule générale $Q^+A^-$, dans laquelle $Q^+$ représente un cation et $A^-$ représente un anion, et d'un catalyseur comprenant au moins un complexe du cobalt avec au moins un ligand L choisi parmi les bases de Lewis ;
- une étape de dépressurisation ;
- une étape de décantation ;
- et une étape de recyclage ;

ledit procédé étant caractérisé en ce que l'addition du ligand L, éventuellement en mélange avec un solvant organique, est réalisée dans une étape post-réactionnelle, et en ce que le rapport molaire entre le ligand L et le composé du cobalt (L/Co) dans cette étape post-réactionnelle est supérieur à 2:1 et de préférence inférieur à 100:1.

Les composés oléfiniques insaturés susceptibles d'être hydroformylés sont choisis parmi les monooléfines, les dioléfines et en particulier les dioléfines conjuguées, les composés oléfiniques comportant un ou plusieurs hétéroatomes, notamment insaturés comme la fonction cétone ou acide carboxylique.

À titre d'exemples non limitatifs, on peut citer l'hydroformylation des pentènes en hexanal et méthylpentanal, des hexènes en isoheptanals, des isooctènes en isononanals, des isodécènes en iso-undécanals, des coupes $C_{11}$ à $C_{16}$ oléfiniques en aldéhydes $C_{12}$ à $C_{17}$. Ces composés oléfiniques peuvent être mis en oeuvre purs ou dilués par des hydrocarbures saturés ou insaturés. Ils peuvent notamment provenir des procédés de transformation des oléfines, tels que les procédés de dimérisation et d'oligomérisation des oléfines (particulièrement C2-C5), ainsi que de tout autre procédé conduisant à la production d'un mélange d'oléfines. À titre non limitatif, on citera comme charges potentielles à hydroformyler selon le procédé de l'invention, les oléfines issues des procédés Dimersol®, Difasol®, Octol® ou SHOP®.

Ce procédé peut s'appliquer à des charges à hydroformyler constituées de monooléfines majoritairement internes, c'est à dire à des mélanges de monooléfines renfermant au plus 30% de monooléfines terminales.

On citera plus particulièrement les mélanges d'octènes ayant la composition suivante :

- octènes linéaires (2 à 10 % en poids),
- méthylheptènes (50 à 70 % en poids),
- diméthylhexènes (25 à 35 % en poids),
- autres monooléfines (1 à 3% en poids)

et dans lesquels moins de 10% sont des oléfines terminales.

Afin d'améliorer la séparation des produits de réaction et du liquide ionique, il est possible d'utiliser un solvant organique en complément du mélange réactionnel décrit ci-dessus. Cette addition de solvant sera préférentiellement réalisée dans une étape post-réactionnelle. Ce solvant organique est choisi plus particulièrement parmi les hydrocarbures aliphatiques, cycliques ou acycliques, saturés ou insaturés, et les hydrocarbures aromatiques ou aromatiques substitués. Parmi ceux-là, on peut choisir de préférence le solvant organique parmi les n-paraffines et iso-paraffines et les hydrocarbures aliphatiques cycliques. De la façon la plus préférée, le solvant organique pourra consister en le ou les composés oléfiniquement insaturés à transformer. Il pourra être utilisé pour additionner le ligand.

Les composés du cobalt précurseurs du catalyseur sont choisis parmi les sels de cobalt, comme les acétylacétonates, les alcoolates, les carboxylates et en particulier le formiate ou l'acétate, et les complexes carbonyle, comme le dicobaltoctacarbonyle, l'hydrure de cobalt tétracarbonyle et les clusters carbonyles. Le choix du composé précurseur du cobalt n'est pas critique.

Le ligand basique de Lewis est choisi parmi les ligands oxygénés, les ligands soufrés, les ligands azotés et les ligands phosphorés, substitués ou non par des groupements fonctionnels ioniques. Les groupements fonctionnels ioniques sont choisis parmi les sulfonates, les carboxylates, les phosphates, les ammoniums, les phosphoniums, et les imidazoliums.

Les ligands oxygénés sont choisis plus particulièrement parmi les alcools, les phénols, les éthers, les cétones et les acétals. On peut citer à titre d'exemples non limitatifs le méthanol, l'éthanol, le phénol, le diéthyléther, le dibutyléther, le diphényléther, le tétrahydrofurane, le dioxane-1,4, le dioxolane-1,3, le glyme, le diglyme, l'acétone, la méthyéthylcétone, l'acétophénone, le méthylal, le diméthoxy-2,2 propane et le di(éthyl-2 hexyloxy)-2,2 propane.

Les ligands soufrés sont choisis plus particulièrement parmi les thiols, les thiophénols, les thioéthers et les disulfures. On peut citer à titre d'exemples non limitatifs le méthanethiol, l'éthanethiol, le thiophénol, le diéthylsulfure, le diméthyldisulfure et le tétrahydrothiophène.

Les ligands azotés sont choisis plus particulièrement parmi les mono-amines, les di-, tri- et poly-amines, les imines, les diimines, les pyridines, les bipyridines, les imidazoles, les pyrroles et les pyrazoles. Préférentiellement, le ligand de type pyridine est choisi parmi les pyridines non-substituées et les pyridines substituées en position 2,3,4 ou 5 par des groupements alkyles, aryles, aralkyles, alcoxy, aryloxy, hydroxy, halogénures ou carboxyalkyles. On peut citer à titre d'exemples non limitatifs la méthylamine, la triméthylamine, la triéthylamine, l'éthylénediamine, la diéthylènetriamine, le diazabicyclooctane, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-dümine, la N,N'-bis-(diisopropyl-2.6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, la pyridine, la 2-picoline, la 4-picoline, la t-butyl-2-pyridine, la t-butyl-4-pyridine, la butyl-3-pyridine, la phényl-2-pyridine, la phényl-3-pyridine, la phényl-4-pyridine, la benzyl-2-pyridine, la benzyl-4-pyridine, la méthoxy-2-pyridine, la méthoxy-3-pyridine, la méthoxy-4-pyridine, la di(t-butyl)-2,6-pyridine, la 2,2'-bipyridine, la 4,4'-bipyridine, la di(phényl)-2,6-pyridine, la (phényl-3-propyl)-4-pyridine, l'imidazole, le N-méthylimidazole, le N-butylimidazole, le pyrrole, le N-méthylpyrrole et le diméthyl-2,5-pyrrole.

Les ligands phosphorés sont choisis plus particulièrement parmi les phosphines, les polyphosphines et les oxydes de phosphines, les phosphites. On peut citer à titre d'exemples non limitatifs la tributylphosphine, la trisopropylphosphine, la tricyclohexylphosphine, la triphénylphosphine, la tris(o-tolyl)phosphine, le bis(diphénylphosphino)éthane, l'oxyde de trioctylphosphine, l'oxyde de triphénylphosphine et le triphénylphosphite.

Les ligands préférés sont choisis plus particulièrement parmi les dérivés de la pyridine. On peut citer à titre d'exemples non limitatifs le ligand pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-trifluorométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline et la 1,10-phénanthroline.

La composition catalytique est obtenue par mélange, d'une manière quelconque, du liquide ionique avec le composé du cobalt et le ligand. On peut également dissoudre préalablement le composé du métal de transition et/ou le ligand dans un solvant organique.

Le complexe qui se forme entre le précurseur de cobalt et le ligand peut être préparé préalablement à la réaction par mélange du précurseur de cobalt avec le ligand dans un solvant convenable, par exemple un solvant organique, ou dans le liquide ionique non-aqueux qui sera utilisé ensuite dans la réaction catalytique. Le complexe peut aussi être préparé *in situ* par mélange du précurseur de cobalt et du ligand directement dans le réacteur d'hydroformylation.

La concentration du complexe de cobalt dans le liquide ionique non-aqueux n'est pas critique. Elle est avantageusement comprise entre 0,1 mmole (en atomes de cobalt) par litre et 10 moles par litre de liquide ionique, de préférence entre 10 mmoles et 5 moles par litre, et, de manière encore plus préférée, entre 50 mmoles et 1 mole par litre.

Le rapport des pressions partielles de l'hydrogène au monoxyde de carbone utilisé dans le milieu réactionnel pour l'hydroformylation peut être de 10:1 à 1:10, de préférence de 1 :1, mais tout autre rapport peut être utilisé dans la mise en oeuvre du procédé.

La température à laquelle se fera l'hydroformylation sera comprise entre 30°C et 250°C. Elle est avantageusement inférieure à 200°C et de préférence comprise entre 50°C et 180°C. La pression peut être comprise entre 1 MPa et 30 MPa, de préférence entre 2 MPa et 20 MPa.

La réaction catalytique d'hydroformylation des composés insaturés peut être conduite avec un ou plusieurs étages de réaction. Dans une mise en oeuvre en continu, l'effluent du réacteur sous pression est transféré dans une zone où il est dépressurisé jusqu'à une pression inférieure à 1 MPa et de préférence à la pression atmosphérique, à une température au plus égale à 150°C et de préférence inférieure à 60°C. Le contact entre les deux phases liquides peut être maintenu dans cette étape grâce à une agitation mécanique ou à tout autre moyen convenable. Le temps de contact dans la zone de dépressurisation ainsi que les conditions de pression et de température seront choisis de manière adéquate afin d'assurer au mieux le transfert du catalyseur dans la phase liquide ionique non-aqueux.

À la sortie de la zone de dépressurisation, la phase organique contenant les produits de réaction est séparée, avantageusement par simple décantation de la phase liquide ionique non-aqueux contenant la presque totalité du catalyseur. Cette phase liquide ionique qui contient le catalyseur est, au moins en partie, retournée au réacteur, l'autre partie pouvant être traitée pour éliminer des résidus de décomposition du catalyseur. La phase organique peut ensuite être traitée par distillation afin de séparer de manière adéquate les différents composants du système.

Dans la formule $Q^+A^-$ du liquide ionique non-aqueux mis en jeu dans le procédé de l'invention, $Q^+$ représente un cation choisi de préférence parmi les sulfonium quaternaires, les guanidinium quaternaires, les ammonium quaternaires et les phosphonium quaternaires et $A^-$ représente un anion choisi de préférence parmi les anions halogénures, nitrate, sulfate, alkylsulfates, phosphate, alkylphosphates, acétate, halogénoacétates, tétrafluoroborate, tétrachloroborate, hexafluorophosphate, trifluoro-tris-(pentafluoroéthyl)phosphate, hexafluoroantimonate, fluorosulfonate, alkylsulfonates (par exemple le méthylsulfonate), perfluoroalkylsulfonates (par exemple le trifluorométhylsulfonate), bis(perfluoroalkylsulfonyl)amidures (par exemple l'amidure de bis trifluorométhylsulfonyle, de formule $N(CF_3SO_2)_2^-$), le méthylure de tris-trifluorométhylsulfonyle de formule $C(CF_3SO_2)_3^-$, le méthylure de bis-trifluorométhylsulfonyle de formule HC $(CF_3SO_2)2^-$, arènesulfonates, éventuellement substitués par des groupements halogènes ou halogénoalkyles, l'anion tétraphenylborate et les anions tétraphenylborates dont les noyaux aromatiques sont substitués, tétra-(trifluoroacétoxy)-borate, bis-(oxalato)-borate, dicyanamide, tricyanométhylure, ainsi que l'anion tétrachloroaluminate.

Les cations $Q^+$ sont de préférence choisis parmi les sulfonium quaternaires, les guanidinium quaternaires, les ammonium quaternaires et les phosphonium quaternaires.

Dans les formules ci-après, $R^1$, $R^2$, $R^3$, $R^4$, $R^5$ et $R^6$ représentent chacun l'hydrogène (à l'exception du cation $NH_4^+$ pour $NR^1R^2R^3R^{4+}$), de préférence un seul substituant représentant l'hydrogène, ou des radicaux hydrocarbyles ayant de 1 à 30 atomes de carbone, par exemple des groupements alkyles, saturés ou non-saturés, cycloalkyles ou aromatiques, aryles ou aralkyles, éventuellement substitués, comprenant de 1 à 30 atomes de carbone.

$R^1$, $R^2$, $R^3$, $R^4$, $R^5$ et $R^6$ peuvent également représenter des radicaux hydrocarbyles portant une ou plusieurs fonctions choisies parmi les fonctions $-CO_2R$, -C(O)R, -OR, -C(O)NRR', -C(O)N(R)NR'R", -NRR', -SR, -S(O)R, $-S(O)_2R$, $-SO_3R$, -CN, -N(R)P(O)R'R', -PRR', -P(O)RR', -P(OR)(OR'), -P(O)(OR)(OR') dans lesquelles R, R' et R", identiques ou différents, représentent chacun l'hydrogène ou des radicaux hydrocarbyles ayant de 1 à 30 atomes de carbone.

Les cations sulfonium quaternaires et guanidinium quaternaires répondent de préférence à l'une des formules générales :

$SR^1R^2R^{3+}$

et

$C(NR^1R^2)(NR^3R^4)(NR^5R^6)^+$

où $R^1$, $R^2$, $R^3$, $R^4$, $R^5$ et $R^6$, identiques ou différents, sont définis comme précédemment.

Les cations $Q^+$ ammonium et/ou phosphonium quaternaires répondent de préférence à l'une des formules générales $NR^1R^2R^3R^{4+}$ et $PR^1R^2R^3R^{4+}$, ou à l'une des formules générales $R^1R^2N{=}CR^3R^{4+}$ et $R^1R^2P{=}CR^3R^{4+}$ dans lesquelles $R^1$,$R^2$, $R^3$ et $R^4$, identiques ou différents, sont définis comme précédemment.

Les cations ammonium et/ou phosphonium quaternaires peuvent également être dérivés d'hétérocycles azotés et/ou phosphorés comportant 1, 2 ou 3 atomes d'azote et/ou de phosphore, de formules générales :

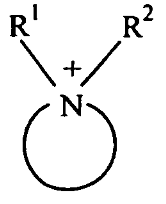
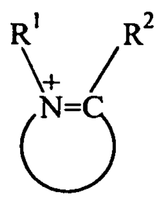
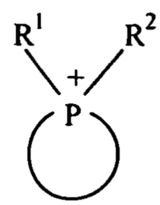
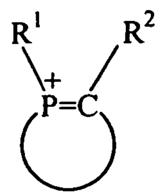

dans lesquelles les cycles sont constitués de 4 à 10 atomes, de préférence de 5 à 6 atomes, et $R^1$ et $R^2$ identiques ou différents, sont définis comme précédemment.

Le cation ammonium ou phosphonium quaternaire peut en outre répondre à l'une des formules générales :

$R^1R^2{+}N{=}CR^3{-}R^7{-}R^3C{=}N{+}R^1R^2$

et

$R^1R^2{+}P{=}CR^3{-}R^7{-}R^3C{=}P{+}R^1R^2$

dans lesquelles $R^1$, $R^2$ et $R^3$ identiques ou différents, sont définis comme précédemment, et $R^7$ représente un radical alkylène ou phenylène.

Parmi les groupements $R^1$ $R^2$ et $R^3$, on mentionnera les radicaux méthyle, éthyle, propyle, isopropyle, butyle primaire, butyle secondaire, butyle tertiaire, amyle, phényle ou benzyle ; $R^7$ pourra être un groupement méthylène, éthylène, propylène ou phénylène.

De manière préférée, le cation ammonium et/ou phosphonium quaternaire $Q^+$ est choisi parmi le N-butylpyridinium, le N-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imidazolium, l' éthyl-3-diméthyl-1,2-imidazolium, le N-butylimidazolium, le N-éthylimidazolium, le cation (hydroxy-2-éthyl)-1-méthyl-3-imidazolium, le cation (carboxy-2-éthyl)-1-méthyl-3-imidazolium, le diéthylpyrazolium, le N-butyl-N-méthylpyrrolidinium, N-éthyl-N-méthylpyrrolidinium, le N-butyl-N-méthylmorpholinium, le triméthylphénylammonium, le triméthylpropylammonium, le triéthylammonium, le tétrabutylphosphonium et le tributyl-tétradécyl-phosphonium.

À titre d'exemples de sels utilisables selon l'invention, on peut citer le bis(trifluorométhylsulfonyl)amidure de butyl-3-méthyl-1-imidazolium, le bis(trifluorométhylsulfonyl)-amidure d'éthyl-3-méthyl-1-imidazolium, le bis(trifluorométhylsulfonyl)amidure de triéthylammonium, le bis(trifluorométhylsulfonyl)amidure de triméthylpropylammonium, le bis (trifluorométhylsulfonyl)amidure de butylimidazolium, le bis(trifluorométhylsulfonyl)-amidure de butyl-3-diméthyl-1,2-imidazolium, le bis(trifluorométhylsulfonyl)amidure de N-butyl-N-méthylpyrrolidinium, le bis(trifluorométhylsulfonyl)amidure de N-éthyl-N-méthylpyrrolidinium, le tétrafluoroborate de butyl-3-méthyl-1-imidazolium, le tétrafluoroborate de butyl-3-diméthyl-1,2-imidazolium, le tétrafluoroborate d'éthyl-3-méthyl-1-imidazolium, l'hexafluophosphate de butyl-3-méthyl-1-imidazolium, l'hexafluoroantimonate de butyl-3-méthyl-1-imidazolium, le trifluoroacétate de butyl-3-méthyl-1-imidazolium, le triflate de éthyl-3-méthyl-1-imidazolium, le triflate de butyl-3-méthyl-1-imidazolium, le méthylsulfate de butyl-3-méthyl-1-imidazolium, le butylsulfate de butyl-3-méthyl-1-imidazolium, le bis(trifluorométhylsulfonyl)amidure de (hydroxy-2-éthyl)-1-méthyl-3-imidazolium, le bis(trifluorométhylsulfonyl)amidure de (carboxy-2-éthyl)-1-méthyl-3-imidazolium et le bis(trifluorométhylsulfonyl)amidure de N-butyl-N-méthylmorpholinium. Ces sels peuvent être utilisés seuls ou en mélange.

Le procédé d'hydroformylation tel que défini dans la description qui précède, peut être mis en oeuvre grâce à une installation comportant (Figure 1) :

- au moins un réacteur A1 ;
- optionnellement un mélangeur B3 ;
- au moins une enceinte de dépressurisation (« dépressurisateur ») B1 ;
- et au moins un décanteur B2 pour la décantation de la phase polaire contenant au moins le solvant ionique non-aqueux et au moins le catalyseur, qui est recyclé au réacteur A1 ;
- dans une section de séparation, au moins une colonne A2 pour la séparation des produits bruts de la réaction et du composé oléfiniquement insaturé à hydroformyler qui n'a pas réagi ainsi que le ligand présent dans la phase organique ;

ainsi que :

- au moins une conduite 1 pour l'introduction de la charge à hydroformyler et du mélange monoxyde de carbone/hydrogène ;
- au moins une conduite 2 pour le transfert de l'effluent du réacteur vers le dépressurisateur B1 ;
- au moins une conduite 3 pour envoyer vers le décanteur B2 le mélange de l'effluent organique et du solvant ionique contenu dans le dépressurisateur B1 ;
- au moins une conduite 4 pour renvoyer vers l'entrée du réacteur A1 les gaz issus du dépressurisateur B1 ;
- au moins une conduite 5 permettant de renvoyer dans le réacteur A1 la phase polaire contenant au moins le liquide ionique et le catalyseur séparée dans B2 ;
- au moins une conduite 6 permettant de soutirer du décanteur B2 les produits bruts de la réaction ;
- au moins une conduite 7 pour recycler vers le dépressurisateur B1 le composé oléfiniquement insaturé à hydroformyler qui n'a pas réagi, ainsi que le ligand séparé dans la colonne A2 (Ce recycle pourra optionnellement être réalisé dans le mélangeur B3 situé en amont du dépressurisateur B1 via une ligne 7') ;
- au moins une conduite 8 permettant d'envoyer les produits sortant en pied de la colonne A2 dans la suite du train de fractionnement des produits ; et
- au moins une conduite 9 permettant de purger le distillat si besoin.
- au moins une conduite 10 pour effectuer l'addition post-réactionnelle du ligand tel que décrit dans l'invention. Le ligand pourra être additionné seul ou en mélange dans un solvant organique;
- optionnellement une conduite 10' pour effectuer l'addition post-réactionnelle du ligand dans le mélangeur B3 situé en amont du dépressurisateur B1.

On comprendra mieux le procédé de l'invention et l'installation à partir de la description qui en est faite ci-après, en liaison avec la Figure 1.

Selon la Figure 1, la réaction est réalisée dans le réacteur A1 en présence de la charge à hydroformyler, qui peut être introduite par la ligne 1, du (ou des) composé(s) de cobalt, de monoxyde de carbone et d'hydrogène, qui peuvent également être introduits par la ligne 1, et en présence d'au moins un liquide ionique non-aqueux. Le liquide ionique peut être introduit dans le réacteur au début de la réaction. Optionnellement, du liquide ionique frais peut être injecté dans le réacteur A1 au cours de la réaction et du liquide ionique usé peut être soutiré de A1 (les moyens d'injection et de soutirage du liquide ionique ne sont pas représentés sur la Figure 1).

La chaleur de réaction est éliminée par les techniques connues de l'homme du métier non représentées sur la Figure 1.

À la sortie de la section réactionnelle, l'effluent du réacteur est envoyé, par la ligne 2, dans au moins un dépressurisateur B1 dans lequel la pression est abaissée. Optionnellement, l'effluent du réacteur peut passer par un mélangeur B3 situé en amont du dépressurisateur B1. Une agitation peut être maintenue dans B1, ainsi que dans B3, soit mécaniquement, soit par tout autre moyen convenable. Les gaz libérés par la dépressurisation s'échappent par la ligne 4 et sont renvoyés vers l'entrée du réacteur A1, après avoir été recomprimés. Lors de cette phase de dépressurisation, du ligand ainsi que les oléfines non transformées et le solvant éventuel peuvent être additionnés au mélange réactionnel directement dans le dépressurisateur B1 par la ligne 7. Ils peuvent alternativement être introduits dans le mélangeur B3 situé en amont du dépressurisateur B1 par la ligne 7'.

L'effluent du dépressurisateur B1 est ensuite envoyé dans le décanteur B2 par la ligne 3. Dans ce décanteur B2, la phase inférieure polaire, qui contient au moins le liquide ionique et le catalyseur, est séparée du mélange des produits et du solvant organique et est renvoyée au réacteur A1 par la ligne 5.

La phase organique supérieure séparée dans le décanteur B2 est envoyée dans une colonne de distillation A2 par la ligne 6. Dans la colonne A2, on sépare en tête le composé oléfiniquement insaturé à hydroformyler qui n'a pas réagi, ainsi que le ligand et le solvant éventuel. Comme décrit ci-dessus, ils sont recyclés au dépressurisateur B1 par la ligne 7 ou, optionnellement, en amont de celui-ci dans le mélangeur B3 par la ligne 7'. Les produits bruts de la réaction recueillis en pied de A2 sont envoyés dans un train de fractionnement spécifique (non représenté) par la ligne 8.
Une autre installation pour la mise en oeuvre du procédé d'hydroformylation tel que défini dans la description qui précède pourra également se présenter selon la Figure 2. Par exemple, cette installation sera particulièrement adaptée pour un système dont les points d'ébullition des différents composants suivent l'ordre croissant suivant (Bp = point d'ébullition) :

$$Bp_{\text{oléfines à transformer}} < Bp_{\text{ligand}} \leq Bp_{\text{solvant}} < Bp_{\text{produits de réaction}}.$$

Cette installation comprend :

- au moins un réacteur A1 ;
- optionnellement un mélangeur B3;
- au moins une enceinte de dépressurisation (« dépressurisateur ») B1;
- et au moins un décanteur B2 pour la décantation de la phase polaire contenant au moins le solvant ionique non-aqueux et au moins le catalyseur qui est recyclé au réacteur A1 ;
- dans la section de séparation au moins une colonne A2 pour la séparation du composé oléfiniquement insaturé à hydroformyler qui n'a pas réagi ;
- au moins une colonne A3 pour la séparation des produits bruts de la réaction (en pied de colonne), du ligand et du solvant éventuel (en tête de colonne).

ainsi que :

- au moins une conduite 1 pour l'introduction de la charge à hydroformyler et du mélange monoxyde de carbone/hydrogène ;
- au moins une conduite 2 pour le transfert de l'effluent du réacteur vers le dépressurisateur B1 ;
- au moins une conduite 3 pour envoyer vers le décanteur B2 le mélange de l'effluent organique et du solvant ionique contenu dans le dépressurisateur B1;
- au moins une conduite 4 pour renvoyer vers le réacteur A1 les gaz issus du dépressurisateur B1 ;
- au moins une conduite 5 permettant de renvoyer dans le réacteur A1 la phase polaire contenant au moins le liquide ionique et le catalyseur séparée dans B2 ;
- au moins une conduite 6 permettant de soutirer du décanteur B2 les produits bruts de la réaction.
- au moins une conduite 7 pour recycler vers le réacteur A1 le composé oléfiniquement insaturé à hydroformyler qui n'a pas réagi.
- au moins une conduite 9 permettant de purger ce distillat si besoin.
- au moins une conduite 10 pour recycler vers le dépressurisateur B1 le ligand séparé dans la colonne A3 ; Ce recycle pourra optionnellement être réalisé dans un mélangeur B3 situé en amont du dépressurisateur B1 (via 10').
- au moins une conduite 11 permettant d'envoyer les produits sortant en pied de la colonne A3 dans la suite du train de fractionnement des produits ; et
- au moins une conduite 12 pour effectuer l'addition post-réactionnelle du ligand tel que décrit dans l'invention. Le ligand pourra être additionné seul ou en mélange dans un solvant.

On comprendra mieux le procédé de l'invention et l'installation à partir de la description qui en est faite ci-après, en liaison avec la Figure 2.
Selon la Figure 2, la réaction est réalisée dans le réacteur A1 en présence de la charge à hydroformyler, qui peut être introduite par la ligne 1, du (ou des) composé(s) de métal de transition, de monoxyde de carbone et d'hydrogène, qui peuvent être également introduits par la ligne 1, et en présence d'au moins un liquide ionique non-aqueux. Le liquide ionique peut être introduit dans le réacteur au début de la réaction. Optionnellement, du liquide ionique frais peut être injecté dans le réacteur A1 au cours de la réaction et du liquide ionique usé soutiré de A1 (les moyens d'injection et de soutirage du liquide ionique ne sont pas représentés sur la Figure 2).
La chaleur de réaction est éliminée par les techniques connues de l'homme du métier qui ne sont pas représentées sur la Figure 2.
À la sortie de la section réactionnelle, l'effluent du réacteur est envoyé, par la ligne 2, dans au moins un dépressurisateur B1, dans lequel la pression est abaissée. Optionnellement, l'effluent du réacteur peut passer par un

mélangeur B3 situé en amont du dépressurisateur B1. Une agitation peut être maintenue dans B1 comme dans B3, soit mécaniquement, soit par tout autre moyen convenable. Les gaz libérés par la dépressurisation s'échappent par la ligne 4 et sont renvoyés vers le réacteur A1 après avoir été recomprimés. Lors de cette phase de dépressurisation, du ligand provenant de la ligne 10, ainsi que le solvant éventuel, provenant de la colonne A3 (comme cela sera décrit plus loin) peuvent être additionnés au mélange réactionnel directement dans le dépressurisateur B1 par la ligne 10. Alternativement cette addition peut être réalisée dans le mélangeur B3 situé en amont du dépressurisateur, par la ligne 10'.

L'effluent du dépressurisateur B1 est ensuite envoyé dans le décanteur B2 par la ligne 3. Dans ce décanteur B2, la phase polaire, qui contient au moins le liquide ionique et le catalyseur, est séparée du mélange des produits et du solvant organique et est renvoyée au réacteur A1 par la ligne 5.

La phase organique séparée dans le décanteur B2 est envoyée dans une colonne de distillation A2 par la ligne 6. Dans la colonne A2, on sépare en tête le composé oléfiniquement insaturé à hydroformyler qui n'a pas réagi. Il est recyclé au réacteur A1 par la ligne 7 ou optionnellement purgé par la ligne 9. Une partie peut également être recyclée au dépressurisateur par la ligne 7'. Les produits recueillis en pied de A2 sont ensuite envoyés dans une seconde colonne de distillation A3 par la ligne 8. Dans la colonne A3, on sépare en tête le ligand ainsi que le solvant éventuel. Ils sont recyclés au dépressurisateur B1 par la ligne 10 ou optionnellement en amont de celui ci dans le mélangeur B3, par la ligne 10'. Les produits bruts de la réaction recueillis en pied de A3 sont envoyés dans un train de fractionnement spécifique (non représenté) par la ligne 11.

L'Exemple 2 qui suit illustre l'invention sans en limiter la portée. L'Exemple 1 est donné à titre de comparaison.

## EXEMPLE 1 (comparatif)

La réaction d'hydroformylation est conduite dans un autoclave en Hastelloy® d'une contenance de 100 mL, muni d'un collier chauffant permettant la régulation de la température et équipé d'une agitation mécanique efficace (hélices "Rushton" à entraînement gazeux avec contre-pales). Dans cet autoclave purgé au préalable de l'air et de l'humidité et placé sous pression atmosphérique du gaz de synthèse hydrogène-monoxyde de carbone (1/1 molaire), on introduit 0,213 g de dicobalt-octacarbonyle (soit 1,2 mmole de cobalt), 2,0 équivalents molaires de 2-méthoxypyridine (0,273 g), 6 mL de bis(trifluorométhylsulfonyl)amidure de butyl-3-méthyl-1-imidazolium, 15 mL d'heptane et 15 mL de Dimate C8 (charge provenant d'un Dimersol X® : 6 % en poids d'octènes linéaires, 58 % en poids de méthylheptènes et 34 % en poids de diméthylhexènes). On porte la pression du gaz de synthèse à 10 MPa et la température à 130°C et on met l'agitation en route (1000 tr/mn). La pression dans le réacteur est maintenue constante pendant toute la durée de la réaction, dont l'avancement est contrôlé par la mesure de la consommation du gaz de synthèse. Après 6 heures de réaction, on ferme l'arrivée du gaz de synthèse et on laisse refroidir le réacteur jusqu'à 25°C. En maintenant l'agitation (250 tr/mn), on relâche alors lentement la pression jusqu'à la pression atmosphérique. L'agitation est arrêtée et le mélange réactionnel est laissé à décanter pendant une heure. Après soutirage hors de l'autoclave, la phase organique supérieure est légèrement colorée et la phase inférieure orange soutenu.

Afin d'évaluer l'efficacité du recyclage du cobalt, la phase liquide ionique récupérée et isolée ci-dessus est maintenue dans le réacteur qui est rechargé avec 15 mL d'heptane et 15 mL de Dimate C8. Aucune addition de cobalt octacarbonyle ni de 2-méthoxypyridine n'est effectuée. La réaction d'hydroformylation est alors reconduite pendant 6h dans le même appareillage et selon le même mode opératoire que décrit précédemment.

Les résultats de 8 cycles consécutifs sont résumés dans le tableau ci-dessous :

| Cycle | Conversion (%) | Conversion linéaires (%) | Conversion monoramifiés (%) | Conversion diramifiés (%) | Sélectivité aldéhyde (%) |
|---|---|---|---|---|---|
| 0 | 89,0 | 100 | 94,0 | 77,7 | - |
| 1 | 79,6 | 99,7 | 86,5 | 63,4 | - |
| 2 | 71,1 | 98,9 | 79,0 | 52,0 | 78,5 |
| 3 | 67,1 | 98,1 | 74,5 | 48,5 | 80,4 |
| 4 | 57,7 | 95,2 | 65,1 | 38,5 | 82,1 |
| 5 | 52,6 | 92,0 | 59,8 | 33,6 | 90,2 |
| 6 | 46,4 | 85,2 | 52,4 | 29,8 | 88,6 |
| 7 | 37,4 | 78,0 | 43,1 | 21,1 | 92,9 |

**EXEMPLE 2 (selon l'invention)**

La réaction d'hydroformylation est conduite dans le même appareillage et selon le même mode opératoire que décrit dans l'Exemple 1, à ceci près qu'une quantité définie de 2-méthoxypyridine est additionnée après chaque cycle pour assurer un rapport L/Co de 6:1. Suite à cette addition, le système est agité durant 15 mn afin de permettre à tout le cobalt présent en phase organique de retourner dans la phase liquide ionique. Le réacteur est ensuite dépressurisé et le mélange réactionnel est laissé à décanter pendant une heure. Après soutirage hors de l'autoclave, la phase organique supérieure est légèrement colorée et la phase inférieure orange soutenu. La phase liquide ionique ainsi obtenue est réintroduite dans le réacteur, qui est alors rechargé avec 15 mL d'heptane et 15 mL de Dimate C8. La réaction d'hydroformylation est alors reconduite dans le même appareillage et selon le même mode opératoire que décrit dans l'Exemple 1 avec bien sûr l'addition de 2-méthoxypyridine après chaque nouveau cycle.

Les résultats de 8 cycles consécutifs sont résumés dans le tableau ci-dessous :

| Cycle | Conversion (%) | Conversion linéaires (%) | Conversion monoramifiés (%) | Conversion diramifiés (%) | Sélectivité aldéhyde (%) |
|---|---|---|---|---|---|
| 0 | 91,1 | 100,0 | 95,4 | 81,8 | -- |
| 1 | 76,1 | 100,0 | 83,1 | 59,2 | 79,4 |
| 2 | 69,6 | 98,4 | 76,9 | 51,6 | 83,2 |
| 3 | 67,0 | 97,5 | 74,3 | 48,8 | 83,7 |
| 4 | 70,4 | 97,9 | 77,3 | 53,4 | 83,6 |
| 5 | 64,6 | 96,5 | 72,4 | 45,5 | 85,9 |
| 6 | 63,4 | 96,7 | 71,1 | 44,1 | 88,4 |
| 7 | 65,6 | 96,9 | 72,9 | 47,5 | 85,2 |

En conclusion, la comparaison des résultats des Exemples 1 et 2 fait apparaître une stabilité améliorée pour le système mettant en oeuvre le procédé selon l'invention.

## Revendications

1. Procédé pour l'hydroformylation en phase liquide de composés oléfiniquement insaturés qui comprend :

    - une étape réactionnelle, effectuée en présence d'au moins un liquide ionique non-aqueux comprenant au moins un sel de formule générale $Q^+A^-$, dans laquelle $Q^+$ représente un cation et $A^-$ représente un anion, et d'un catalyseur comprenant au moins un complexe du cobalt avec au moins un ligand L choisi parmi les bases de Lewis ;
    - une étape de dépressurisation ;
    - une étape de décantation ;
    - et une étape de recyclage dans laquelle la phase polaire, qui contient au moins le liquide ionique et le catalyseur, séparée du mélange des produits et du solvant organique dans l'étape de décantation est renvoyée à l'étape réactionnelle ;

    ledit procédé étant **caractérisé en ce que** l'addition du ligand L. éventuellement en mélange avec un solvant organique, est réalisée dans une étape post-réactionnelle, et **en ce que** le rapport molaire L/Co dans cette étape post-réactionnelle est supérieur à 2.

2. Procédé selon la revendication 1 dans lequel, dans ladite étape post-réactionnelle, le rapport molaire L/Co est inférieur à 100:1.

3. Procédé selon l'une des revendications 1 ou 2 dans lequel on traite au moins un composé oléfiniquement insaturé susceptible d'être hydroformylé choisi parmi les monooléfines, les dioléfines, les composés oléfiniques comportant un ou plusieurs hétéroatomes

4. Procédé selon l'une des revendications 1 à 3 dans lequel on traite un mélange d'octènes ayant la composition suivante :

- octènes linéaires (2 à 10 % en poids),
- méthylheptènes (50 à 70 % en poids),
- diméthylhexènes (25 à 35 % en poids),
- autres monooléfines (1 à 3% en poids)

et dans lesquels moins de 10% sont des oléfines terminales.

5. Procédé selon l'une des revendications 1 à 4 dans lequel on réalise une addition de solvant organique dans l'étape post-réactionnelle, ledit solvant étant choisi parmi les hydrocarbures aliphatiques, cycliques ou acycliques, saturés ou insaturés, et les hydrocarbures aromatiques ou aromatiques substitués.

6. Procédé selon la revendication 5 dans lequel le solvant organique est choisi parmi les n-paraffines, les iso-paraffines et les hydrocarbures aliphatiques cycliques

7. Procédé selon la revendication 1 à 6 dans lequel le solvant organique consiste en le ou les composés oléfiniquement insaturés à transformer.

8. Procédé selon l'une des revendications 1 à 7 dans lequel les composés du cobalt précurseurs du catalyseur sont choisis parmi les acétylacétonates, les alcoolates, les carboxylates, le dicobalt-oclacarbonyle, l'hydrure de cobalt-tétracarbonyle et les clusters carbonyles

9. Procédé selon l'une des revendications 1 à 8 dans lequel le ligand basique de Lewis est choisi parmi les ligands oxygénés, les ligands soufrés, les ligands azotés et les ligands phosphorés, substitués ou non par des groupements fonctionnels ioniques choisis parmi les sulfonates, les carboxylates, les phosphates, les ammoniums, les phosphoniums et les imidazoliums.

10. Procédé selon la revendication 9 dans lequel le ligand est choisi parmi les alcools, les phénols, les éthers, les cétones, les acétals, les thiols, les thiophénols, les thioéthers et les disulfures, les mono-amines, les di-, tri- et poly-amines, les imines, les diamines, les pyridines, les bipyridines, les imidazoles, les pyrroles et les pyrazoles, les pyridines non-substituées et les pyridines substituées en position 2, 3, 4 ou 5 par des groupements alkyles, aryles, aralkyles, alcoxy, aryloxy, hydroxy, halogénures ou carboxyalkyles, les phosphines, les polyphosphines, les oxydes de phosphines et les phosphites.

11. Procédé selon l'une des revendications 1 à 10 dans lequel le sel de formule générale Q+A- est au moins un sel choisi parmi le bis(trifluorométhylsulfonyl)amidure de butyl-3-méthyl-1-imidazolium, le bis(trifluorométhylsulfonyl)-amidure d'éthyl-3-méthyl-1-imidazolium, le bis(trifluorométhylsulfonyl)amidure de triéthylammonium, le bis(trifluorométhylsulfonyl)amidure de triméthylpropylammonium, le bis(trifluorométhylsulfonyl)amidure de butylimidazolium, le bis(trifluorométhylsulfonyl)amidure de butyl-3-diméthyl-1,2-imidazolium, le bis(trifluorométhylsulfonyl)amidure de N-butyl-N-méthylpyrrolidinium, le bis(trifluorométhylsulfonyl)amidure de N-éthyl-N-méthylpyrrolidinium, le tétrafluoroborate de butyl-3-méthyl-1-imidazolium, le tétrafluoroborate de butyl-3-diméthyl-1,2-imidazolium, le tétrafluoroborate d'éthyl-3-méthyl-1-imidazolium, fhexafluophosphate de butyl-3-méthyl-1-imidazolium, l'hexafluoroantimonate de butyl-3-méthyl-1-imidazolium, le trifluoroacétate de butyl-3-méthyl-1-imidazolium, le triflate de éthyl-3-méthyl-1-imidazolium, le triflate de butyl-3-méthyt-1-imidazolium, le méthylsulfate de butyl-3-méthyl-1-imidazolium, le butylsulfate de butyl-3-méthyl-1-imidazolium, le bis(trifluorométhylsulfonyl)amidure de (hydroxy-2-éthyl)-1-méthyl-3-imidazolium, le bis(trifluorométhylsulfpnyl)amidure de (carboxy-2-éthyl)-1-méthyl-3-imidazolium et le bis (trifluorométhylsulfonyl)amidure de N-butyl-N-méthylmorpholinium, utilisés seuls ou en mélange.

12. Procédé selon l'une des revendications 1 à 11 dans lequel la concentration du complexe de cobalt dans le liquide ionique est comprise entre 0,1 mmole par litre et 10 moles par litre.

13. Procédé selon l'une des revendications 1 à 12 dans lequel la réaction d'hydroformytation est effectuée avec un rapport des pressions partielles de l'hydrogène au monoxyde de carbone de 10:1 à 1:10, à une température comprise entre 30°C et 250°C, sous une pression comprise entre 1 MPa et 30 MPa.

## Claims

**1.** A method for hydroformylating in the liquid phase olefinically unsaturated compounds, comprising:

- a reaction stage carried out in the presence of at least one non-aqueous ionic liquid comprising at least one salt of general formula $Q^+A^-$, wherein $Q^+$ represents a cation and $A^-$ represents an anion, and of a catalyst comprising at least one cobalt complex with at least one ligand L selected from among the Lewis bases,
- a depressurization stage,
- a decantation stage,
- and a recycling stage wherein the polar phase, which contains at least the ionic liquid and the catalyst, separated from the mixture of products and from the organic solvent in the decantation stage, is sent back to the reaction stage,

said method being **characterized in that** the addition of ligand L, possibly in admixture with an organic solvent, is carried out in a post-reaction stage, and **in that** the molar ratio L/Co in this post-reaction stage is above 2.

**2.** A method as claimed in claim 1 wherein, in said post-reaction stage, molar ratio L/Co is below 100:1.

**3.** A method as claimed in any one of claims 1 or 2, wherein at least one olefinically unsaturated compound likely to be hydroformylated, selected from among the mono-olefins, diolefins, olefinic compounds comprising one or more heteroatoms, is treated.

**4.** A method as claimed in any one of claims 1 to 3, wherein an octene mixture having the following composition as follows

- linear octenes (2 to 10 % by weight),
- methylheptenes (50 to 70 % by weight),
- dimethylhexenes (25 to 35 % by weight),
- other mono-olefins (1 to 3 % by weight)

wherein less than 10 % are terminal mono-olefins is treated.

**5.** A method as claimed in any one of claims 1 to 4, wherein organic solvent addition is performed in a post-reaction stage, said solvent being selected from among the aliphatic hydrocarbons, cyclic or acyclic, saturated or unsaturated, and the aromatic or substituted aromatic hydrocarbons.

**6.** A method as claimed in claim 5 wherein the organic solvent is selected from among the n-paraffins, iso-paraffins and the cyclic aliphatic hydrocarbons.

**7.** A method as claimed in any one of claims 1 to 6, wherein the organic solvent consists of the olefinically unsaturated compound(s) to be converted.

**8.** A method as claimed in any one of claims 1 to 7, wherein the catalyst precursor cobalt compounds are selected from among the acetylacetonates, alcoholates, carboxylates, dicobalt-octacarbonyl, cobalt-tetracarbonyl hydride and carbonyl clusters.

**9.** A method as claimed in any one of claims 1 to 8, wherein the basic Lewis ligand is selected from among the oxygen-containing ligands, the sulfur-containing ligands, the nitrogen-containing ligands and the phosphorus-containing ligands, substituted or not by ionic functional groups selected from among the sulfonates, carboxylates, phosphates, ammoniums, phosphoniums and imidazoliums.

**10.** A method as claimed in claim 9, wherein the ligand is selected from among the alcohols, phenols, ethers, ketones, acetals, thiols, thiophenols, thioethers and disulfides, mono-amines, di-, tri- and poly-amines, imines, di-imines, pyridines, bipyridines, imidazoles, pyrroles and pyrazoles, the non-substituted pyridines and the pyridines substituted in position 2, 3, 4 or 5 by alkyl, aryl, aralkyl, alcoxy, aryloxy, hydroxy, halogenide or carboxyalkyl groups, the phosphines, polyphosphines, phosphine oxides and phosphites.

**11.** A method as claimed in any one of claims 1 to 10, wherein the salt of general formula Q+A- is at least a salt selected from among butyl-3-methyl-1-imidazolium bis(trifluoromethylsulfonyl)amidide, ethyl-3-methyl-1-imidazolium bis(trifluoromethylsulfonyl)amidide, triethylammonium bis(trifluoromethyl-sulfonyl)amidide, trimethylpropylammonium bis (trifluoromethylsulfonyl)amidide, butylimidazolium bis(trifluoromethylsulfonyl)amidide, butyl-3-dimethyl-1,2-imidazolium bis(trifluoromethylsulfonyl)amidide, N-butyl-N-methylpyrrolidinium bis(trifluoromethylsulfonyl)-amidide, N-ethyl-N-methylpyrrolidinium bis(trifluoro-methylsulfonyl)amidide, butyl-3-methyl-1-imidazolium tetrafluoroborate, butyl-3-dimethyl-1,2-imidazolium tetrafluoroborate, ethyl-3-methyl-1-imidazolium tetrafluoroborate, butyl-3-methyl-1-imidazolium hexafluophosphate, butyl-3-methyl-l-imidazolium hexafluoroantimonate, butyl-3-methyl-l-imidazolium trifluoroacetate, ethyl-3-methyl-l-imidazolium triflate, butyl-3-methyl-l-imidazolium triflate, butyl-3-methyl-l-imidazolium methylsulfate, butyl-3-methyl-1-imidazolium butylsulfate, (hydroxy-2-ethyl)-1-methyl-3-imidazolium bis(trifluoromethylsulfonyl)amidide, (carboxy-2-ethyl)-1-methyl-3-imidazolium bis(trifluoromethylsulfonyl)amidide and N-butyl-N-methylmorpholinium bis(trifluoromethylsulfonyl)amidide, used alone or in admixture.

**12.** A method as claimed in any one of claims 1 to 11, wherein the concentration of the cobalt complex in the ionic liquid ranges between 0.1 mmole per litre and 10 moles per litre.

**13.** A method as claimed in any one of claims 1 to 12, wherein the hydroformylation reaction is carried out with a ratio of the partial pressures of hydrogen to carbon monoxide of 10:1 to 1:10, at a temperature ranging between 30°C and 250°C, and at a pressure ranging between 1 MPa and 30 MPa.

## Patentansprüche

**1.** Verfahren zur Hydroformylierung von olefinisch ungesättigten Verbindungen in flüssiger Phase, welches Folgendes umfasst

- einen Reaktionsschritt, durchgeführt in Gegenwart mindestens einer ionischen nicht-wässrigen Flüssigkeit, die mindestens ein Salz der allgemeinen Formel $Q^+A^-$ umfasst, wobei $Q^+$ für ein Kation steht und $A^-$ für ein Anion steht, und eines Katalysator, der mindestens einen Kobaltkomplex mit mindestens einem Liganden L, welcher aus den Lewisbasen ausgewählt ist, umfasst;
- einen Schritt der Druckentspannung;
- einen Schritt des Dekantierens;
- und einen Schritt des Rückführens, bei welchem die polare Phase, die mindestens eine ionische Flüssigkeit und den Katalysator enthält und im Dekantierschritt von der Mischung der Produkte und vom organischen Lösemittel abgetrennt wurde, in den Reaktionsschritt zurückgeführt wird;

wobei das Verfahren **dadurch gekennzeichnet ist, dass** der Zusatz des Liganden L, möglicherweise in Mischung mit einem organischen Lösemittel, in einem Schritt durchgeführt wird, welcher der Reaktion nachgeschaltet ist, und **dadurch**, dass das L/Co-Molverhältnis in diesem Schritt, welcher der Reaktion nachgeschaltet ist, größer als 2 ist.

**2.** Verfahren nach Anspruch 1, wobei in dem Schritt, welcher der Reaktion nachgeschaltet ist, das L/Co-Motverhättnis kleiner als 100:1 ist.

**3.** Verfahren nach einem der Ansprüche 1 oder 2, wobei mindestens eine olefinisch ungesättigte Verbindung behandelt wird, die dazu geeignet ist, eine Hydroformylierung zu erfahren, und aus den Monoolefinen, den Diolefinen, den olefinischen Verbindungen, welche ein oder mehrere Heteroatome umfassen, ausgewählt ist.

**4.** Verfahren nach einem der Ansprüche 1 bis 3, wobei eine Octenmischung behandelt wird, welche die folgende Zusammensetzung hat:

- geradkettige Octene (2 bis 10 Gew.-%),
- Methylheptene (50 bis 70 Gew.-%),
- Dimethylhexene (25 bis 35 Gew.-%),
- sonstige Monoolefine (1 bis 3 Gew.-%)

und wobei endständige Olefine mindestens 10 % davon ausmachen.

**5.** Verfahren nach einem der Ansprüche 1 bis 4, wobei in dem Schritt, welcher der Reaktion nachgeschaltet ist, ein Zusatz eines organischen Lösemittels erfolgt, wobei das Lösemittel aus den aliphatischen, cyclischen oder offenkettigen, gesättigten oder ungesättigten Kohlenwasserstoffen und den aromatischen oder aromatischen substituierten Kohlenwasserstoffen ausgewählt ist.

6. Verfahren nach Anspruch 5, wobei das organisches Lösemittel aus den n-Paraffinen, den iso-Paraffinen und den aliphatischen cyclischen Kohlenwasserstoffen ausgewählt ist.

7. Verfahren nach Anspruch 1 bis 6, wobei das organische Lösemittel aus der oder den olefinisch ungesättigten Verbindungen, die umgewandelt werden soll(en), besteht.

8. Verfahren nach einem der Ansprüche 1 bis 7, wobei die Kobaltverbindungen, die als Vorläufersubstanzen des Katalysators dienen, aus den Acetylacetonaten, den Alkoholaten, den Carboxylaten, den di-Kobalt-Octacarbonylen, Kobalt-Tetracarbonyl-Hydrid und den Carbonyl-Clustern ausgewählt sind.

9. Verfahren nach einem der Ansprüche 1 bis 8, wobei der basische Lewis-Ligand aus den sauerstoffhaltigen Liganden, den schwefelhaltigen Liganden, den stickstoffhaltigen Liganden und den phosphorhaltigen Liganden ausgewählt ist, wobei diese mit funktionellen Gruppen substituiert sein können oder nicht, welche aus den Sulfonaten, den Carboxylaten, den Phosphaten, den Ammoniumgruppen, den Phosphoniumgruppen und den Imidazoliumgruppen ausgewählt sind.

10. Verfahren nach Anspruch 9, wobei der Ligand aus den Alkoholen, den Phenolen, den Ethern, den Ketonen, den Acetalen, den Thiolen, den Thiophenolen, den Thioethern, den Disulfiden, den Monoaminen, den Di-, Tri- und Polyaminen, den Iminen, den Diaminen. den Pyridinen, den Bipyridinen, den Imidazolen, den Pyrrolen und den Pyrazolen, den unsubstituierten Pyridinen und den Pyridinen, die an Position 2, 3, 4 oder 5 mit Alkyl-, Aryl- Aralkyl-, Alkoxy-, Aryloxy-, Hydroxyl-, Halogenid oder Carboxyalkylgruppen substituiert sind, den Phosphinen, den Polyphosphinen, den Oxiden von Phosphinen und den Phosphiten ausgewählt ist.

11. Verfahren nach einem der Ansprüche 1 bis 10, wobei es sich bei dem Salz der allgemeinen Formel Q+A- um mindestens ein Salz handelt, das aus 3-Butyl-1-methylimidazolium-bis(trifluormethylsulfonyl)amid, 3-Ethyl-1-methylimidazolium-bis(trifluormethylsulfonyl)amid, Triethylammonium-bis(trifluormethylsulfonyl)amid, Trimethylpropylammonium-bis(trifluormethylsulfonyl)amid, Butylimidazolium-bis(trifluormethylsulfonyl)amid, Butyl-3-dimethyl-1,2-imidazolium-bis(trifluormethylsulfonyl)amid, N-Butyl-N-methylpyrrolidinium-bis(trifluormethylsulfonyl)amid, N-Ethyl-N-methylpyrrolidinium-bis(trifluormethylsulfonyl)amid, 3-Butyl-1-methylimidazoliumtetrafluorborat, 3-Butyl-1,2-dimethylimidazoliumtetrafluorborat, 3-Ethyl-1-methylimidazoliumtetrafluorborat, 3-Butyl-1-methylimidazoliumhexafluorphosphat, 3-Butyl-1-methylimidazoliumhexafluorantimonat, 3-Butyl-1-methylimidazoliumtrifluoracetat, 3-Ethyl-1-methylimidazoliumtriflat, 3-Butyl-1-methylimidazoliumtriflat, 3-Butyl-1-methylimidazoliummethylsulfat, 3-Butyl-1-methylimidazoliumbutylsulfat, 1-(Hydroxy-2-ethyl)-3-methylimidazolium-bis(trifluormethylsulfonyl)amid, 1-(Carboxy-2-ethyl)-3-methylimidazolium-bis(trifluormethylsulfonyl)amid oder N-Butyl-N-methylmorpholinium-bis (trifluormethylsulfonyl)amid ausgewählt ist und allein oder in Mischung verwendet wird.

12. Verfahren nach einem der Ansprüche 1 bis 11, wobei die Konzentration des Kobaltlomplexes in der ionischen Flüssigkeit im Bereich von 0,1 mmol pro Liter und 10 mol pro Liter liegt.

13. Verfahren nach einem der Ansprüche 1 bis 12, wobei die Hydroformylierungsreaktion mit einem Partialdruckverhältnis von Wasserstoff und Kohlenmonoxid, das 10:1 bis 1:10 beträgt, bei einer Temperatur im Bereich von 30 °C bis 250 °C, unter einem Druck im Bereich von 1 MPa bis 30 MPa, durchgeführt wird.

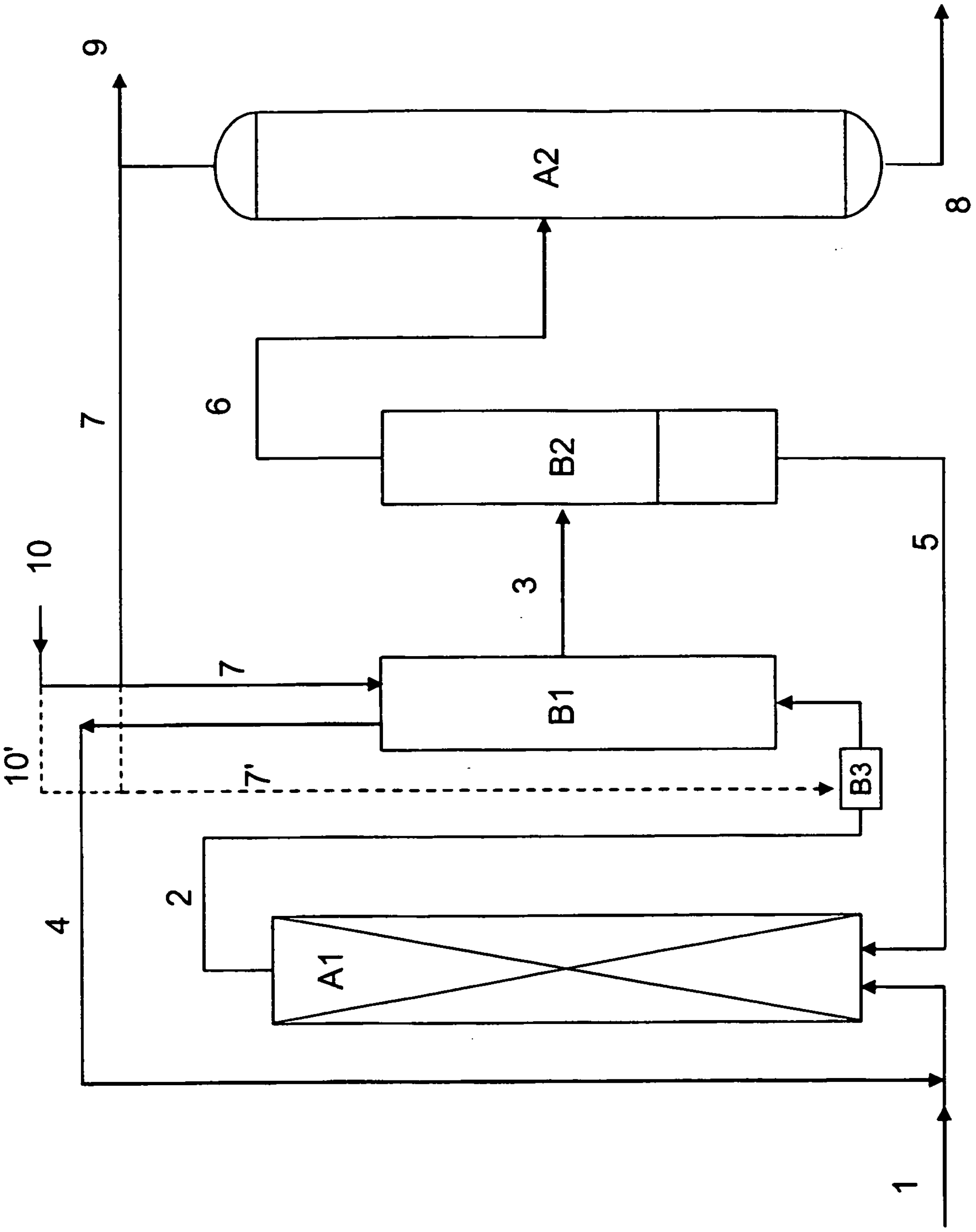


FIG. 1

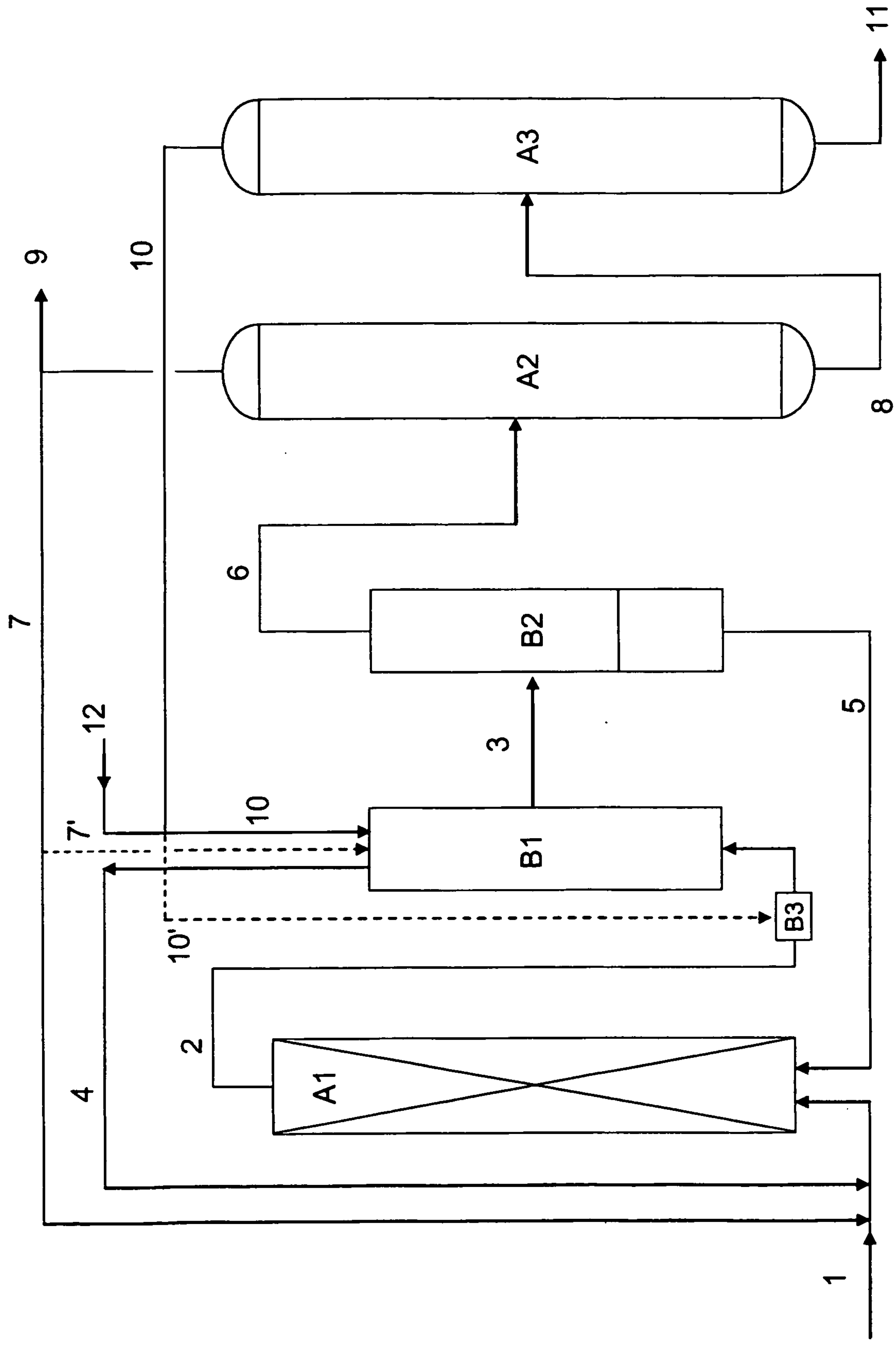


FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5874638 A
- US 6617474 A
- US 20030225303 A


### Littérature non-brevet citée dans la description


- **Bartik et al.** *Organometallics,* 1993, vol. 12, 164-170
- *J. Organomet. Chem.,* vol. 480, 15-21
- **Beller et al.** *J. Molecular Catal. A: Chemical,* 1999, vol. 143, 31-39